# EUROPÄISCHE PATENTSCHRIFT

(11) **EP 1 161 923 B1**
(45) Veröffentlichungstag und Bekanntmachung des Hinweises auf die Patenterteilung: **14.12.2005**
(21) Anmeldenummer: 01113851.8
(22) Anmeldetag: 07.06.2001
(51) Int. Cl.: A61B 5/15

(54) **Behältnis für die Entnahme von Proben und insbesondere Blutproben**
Container for taking samples and in particular blood samples
Récipients de prise d'échantillons et en particulier d'échantillons de sang

(30) Priorität: 08.06.2000 DE 10028482
(43) Veröffentlichungstag der Anmeldung: 12.12.2001
(73) Patentinhaber: KABE Labortechnik Gesellschaft mit beschränkter Haftung, D-51588 Nümbrecht-Elsenroth (DE)
(72) Erfinder: Kolpe, Dieter, 51674 Wiehl (DE)
(74) Vertreter: Christophersen & Partner

(56) Entgegenhaltungen:
- EP-A- 0 510 683
- EP-A- 0 643 944
- WO-A-98/44970
- DE-A- 4 021 355
- DE-A- 4 402 690
- GB-A- 1 036 000
- US-A- 5 779 074

## Beschreibung

Die vorliegende Erfindung betrifft Behältnis für die Entnahme von Proben und insbesondere Blutproben, bestehend aus einem Röhrchen und einer zum Verschließen auf die Öffnung des Röhrchens aufsetzbaren Kappe, die sich mit einem Absatz gegenüber der die Öffnung umgebenden Stirnfläche des Röhrchens abstützt und mit einem Bund den Außenrand des Röhrchens umschließt, wobei in die Kappe ein gegenüber der Kappe abgedichteter Stopfen aus durchstechbarem und sich selbsttätig wieder verschließendem Material eingesetzt ist.

Aus der DE 196 47 978 A1 ist eine Blutentnahmevorrichtung mit einem solcher Art ausgebildeten Behältnis bekannt. Auf einen an seinem vorderen Ende mit einer Kanüle zum Anstechen der Vene versehenen Nadelhalter ist ein durch einen Stopfen verschlossenes Entnahmeröhrchen von hinten her aufsetzbar. Während des Aufsetzens durchsticht eine rückwärtig aus dem Nadelhalter herausragende Kanüle den Stopfen, so daß anschließend eine direkte Verbindung zwischen dem Volumen des Entnahmeröhrchens und der Vene besteht und Blut in das Entnahmeröhrchen einströmen kann. Das Entnahmeröhrchen ist an seinem vorderen Ende mit einer Verschlußkappe versehen, die den Stopfen aufnimmt.

Das Entnahmeröhrchen nach der DE 196 47 978 A1 arbeitet nach dem Aspirationsprinzip, d. h. es befindet sich darin ein Kolben mit Kolbenstange, so daß nach dem Ansetzen an den Nadelhalter durch Herausziehen der Kolbenstange mit dem Kolben das Blut infolge des erzeugten Unterdrucks in das Entnahmeröhrchen gelangt. Für derartige, nach dem Aspirationsprinzip arbeitende Blutentnahmeröhrchen ist die bei der bekannten Blutentnahmevorrichtung verwirklichte Abdichtung der Kappe gegenüber dem Röhrchen durch Dichtwülste der Kappe, die sich von Innen an die Innenwand des Röhrchens anlegen, ausreichend. Hingegen wäre die in der genannten Druckschrift beschriebene Abdichtung nicht ausreichend, wenn ein Blutentnahme-Behältnis nach dem Vakuumprinzip verwendet würde. In dessen Inneren wird herstellerseitig ein Unterdruck erzeugt, wodurch nach dem Durchstechen des in der Kappe angeordneten Stopfens infolge des Unterdrucks ein gleichsam automatisches Ansaugen des Blutes aus der Vene erfolgt. Ein nach dem Vakuumprinzip arbeitendes Blutentnahme-Behältnis ist z. B. in der DE-OS 18 12 742 beschrieben. Der Verschluß des Vakuumröhrchens erfolgt dabei über einen auf die Öffnung aufgesetzten und sich auf der Stirnfläche des Röhrchens abstützenden Pfropfen. Dieser ist in seinem Zentrum mit einer Materialverdünnung versehen, welche beim Ansetzen in den Nadelhalter von dessen Kanüle durchstochen wird. Allerdings besteht bei Vakuumröhrchen der in der DE-OS 18 12 742 beschriebenen Art die Gefahr, daß beim Abziehen des Röhrchens nach der Entnahme des Blutes infolge der Reibung zwischen Pfropfen und Kanüle der Pfropfen an der Kanüle hängen bleibt und dadurch aus dem Röhrchen herausgezogen wird, wodurch dessen Inhalt herausfließen kann.

Aus der GB-A-1 036 000 ist ein Behältnis mit den Merkmalen des ersten Teils des Patentanspruchs 1 bekannt.

Der Erfindung liegt die **Aufgabe** zugrunde, ein funktionssicheres und aus wenigen Einzelteilen einfach zusammensetzbares Behältnis für die Entnahme von Proben und insbesondere Blutproben zu schaffen, bei dem der Stopfen auch im Falle eines starken Unterdrucks innerhalb des Röhrchens nicht in dieses hineingezogen wird.

Zur **Lösung** wird bei einem Behältnis für die Entnahme von Proben der eingangs genannten Art vorgeschlagen, daß das Behältnis die Merkmale des zweiten Teils des Anspruchs 1 aufweist.

Der Stopfen übt daher eine doppelte Funktion aus. Zum einen bildet er einen Verschluß des Röhrchens, welcher durch die rückseitig an dem Nadelhalter der Blutentnahmevorrichtung angeordnete Kanüle durchstechbar ist, wobei nach dem Herausziehen der Kanüle infolge des sich selbsttätig wieder verschließenden Materials des Stopfes wiederum ein vollständiger Verschluß des Röhrchens erfolgt. Hierbei wird der Stopfen durch sein Anlegen einerseits an der Kappe und andererseits an dem Röhrchen fixiert, so daß er beim Herausziehen von dem Nadelhalter nicht versehentlich mit abgezogen werden kann.

Die zweite Funktion des Stopfens besteht in einer Abdichtung der Kappe gegenüber dem Röhrchen, indem an dem Stopfen einstückig ein umlaufender Kragen angeformt ist. Dabei wird der Umstand zunutze gemacht, daß jene Elastizität, welche die Rückverformung des durchstechbaren Stopfens bewirkt, einhergeht mit einer guten Dichtwirkung des Materials. Erreicht wird dies durch ein relativ weiches und elastisches Material für den Stopfen, z. B. ein Kautschuk oder ein thermoplastisches Elastomer.

Vorzugsweise befindet sich der Kragen zwischen der Stirnfläche des Röhrchens und dem Absatz der Kappe. In diesem Bereich erfolgt eine axiale Abdichtung der Kappe gegenüber dem Röhrchen, wobei die für die Erzielung der Dichtwirkung erforderlichen Axialkräfte von der Kappe aufgebracht werden können, indem diese in geeigneter Weise mit dem Röhrchen verrastet wird. Sofern das Röhrchen nach dem Vakuumprinzip arbeitet, sorgt der Unterdruck in dem Inneren des Röhrchens bereits für die erforderliche Axialkraft, mit der der umlaufende Kragen zwischen der Stirnfläche des Röhrchens und dem Absatz der Kappe eingeklemmt wird.

Die Abdichtwirkung läßt sich noch einmal verbessern, indem sich der Kragen radial bis an die Innenseite des an der Kappe angeformten Bundes erstreckt. Die auf den Kragen ausgeübten axialen Druckkräfte fügen wegen der plastischen Verformbarkeit des Materials des Stopfens zu einer leichten radialen Aufweitung des Kragens, so daß sich dessen Umfangsfläche mit der Wirkung einer zusätzlichen Abdichtung gegen die Innenseite des an der Kappe angeformten Bundes drückt.

Gemäß der Erfindung wird vorgeschlagen, daß die Kappe einen von dem Absatz ausgehenden, im wesentlichen zylindrischen Längsabschnitt aufweist, dessen Außendurchmesser geringer als der Außendurchmesser des Bundes sein kann, und daß sich der durchstechbare Teil des Stopfens innerhalb dieses Längsabschnittes befindet. Indem der äußere Teil der Kappe einen gegenüber dem Durchmesser des Bundes sowie dem Durchmesser des Röhrchens verringerten Außendurchmesser aufweist, läßt sich auch der Nadelhalter, in den das Behältnis zur Blutentnahme einsetzbar ist, insgesamt schlanker gestalten. Die schlankere Gestaltung des Nadelhalters wiederum ermöglicht es dem Arzt in vorteilhafter Weise, die Nadel unter einem flacheren Winkel in die Vene des Patienten einzuführen.

Mit der Erfindung wird ferner vorzugsweise vorgeschlagen, daß die Kappe mit um den zylindrischen Längsabschnitt herum angeordneten Verriegelungselementen versehen ist, mittels deren sich das Röhrchen formschlüssig an einem Nadelhalter verriegeln läßt, wobei der zylindrische Längsabschnitt von einer ebenfalls zylindrischen Führungshülse des Nadelhalters aufgenommen wird.

Desweiteren können auf der Innenseite des Bundes Rastmittel ausgebildet sein die in korrespondierende Rastmittel auf dem Außenrand des Röhrchens eingreifen.

Um zu verhindern, daß bei starkem Unterdruck in dem Röhrchen der Stopfen in das Innere des Röhrchens hineingezogen wird, wird mit der Erfindung vorgeschlagen, daß auf der Innenseite des zylindrischen Längsabschnitts in Höhe des durchstechbaren Teils des Stopfens Rückhalteelemente ausgebildet sind, die den Stopfen in axialer Richtung formschlüssig fixieren. Von Vorteil ist es in diesem Fall, wenn der Stopfen in Höhe der Rückhalteelemente mit einer die Rückhalteelemente aufnehmenden Umfangsnut versehen ist, und die Rückhalteelemente jeweils eine steile und eine flache, rampenförmige Flanke aufweisen, wobei die steilen Flanken zu der Öffnung der Kappe hin weisen. Auf diese Weise läßt sich bei der Herstellung des Behältnisses der Stopfen sehr einfach in die Kappe hinein drücken und in dieser Stellung an den Rückhalteelementen des zylindrischen Längsabschnitts der Kappe verriegeln. Die Rückhalteelemente wirken hierbei wie Widerhaken und lassen ein Zurückgleiten des Stopfen innerhalb des zylindrischen Abschnittes auch im Falle eines starken Unterdrucks in dem Röhrchen nicht mehr zu.

Weitere Einzelheiten und Vorteile werden nachfolgend anhand eines Beispieles und unter Bezugnahme auf die Zeichnung erläutert. Darin zeigen:
- Fig. 1: in einem Längsschnitt den Nadelhalter einer Blutentnahmevorrichtung;
- Fig. 2: in einem Längsschnitt den vorderen Teil eines Entnahmeröhrchens der Blutentnahmevorrichtung;
- Fig. 3: in einer Schnittdarstellung die Gegenstände nach den Fign. 1 und 2 in zusammengesetztem Zustand und
- Fig. 4: eine der Fig. 3 entsprechende Darstellung, allerdings unter Verwendung eines Entnahmeröhrchens nach dem Aspirationsprinzip anstelle des in den Fign. 2 und 3 dargestellten Entnahmeröhrchens nach dem Vakuumprinzip.

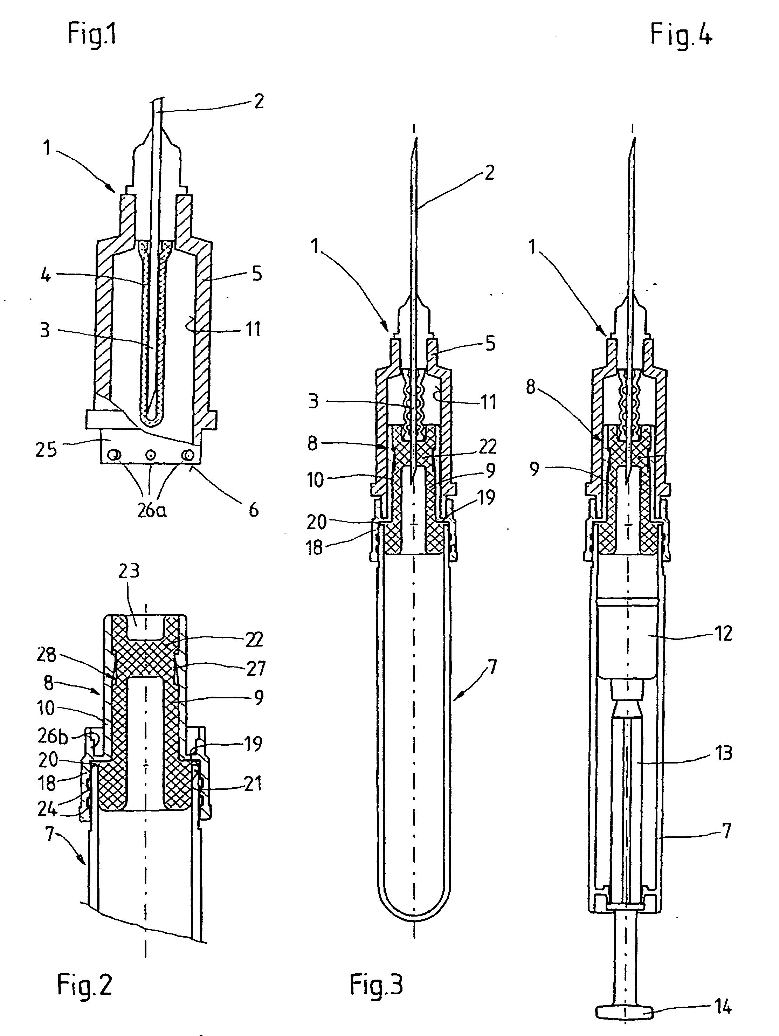

Fig. 1 zeigt einen Nadelhalter 1 einer Blutentnahmevorrichtung, aus dessen vorderem Ende eine Nadel 2 zum Anstechen der Vene des Patienten herausragt. Ebenso wie an ihrem vorderen Anstechende ist die Nadel 2 auch an ihrem hinteren Ende angespitzt und bildet auch dort eine Kanüle 3. Über die Kanüle 3 ist ein flexibler Gummischlauch 4 gezogen, so daß die Kanüle 3 einschließlich ihrer Spitze durch den Gummischlauch 4 ummantelt und abgedeckt ist.

Bestandteil des Nadelhalters 1 ist ferner eine sich nach hinten erstreckende und die Kanüle 3 mit Abstand umgebende Hülse 5 aus Kunststoff. Die hintere Stirnfläche 6 der Hülse 5 erstreckt sich dabei weiter nach hinten, als die Spitze der Kanüle 3, so daß Verletzungen bei der Handhabung des Nadelhalters 1 vermieden werden. Auf ihrer Außenfläche ist die Hülse 5 mit einer die Griffigkeit erhöhenden Riffelung versehen.

Fig. 2 zeigt ein Entnahmeröhrchen 7, welches auf den in Fig. 1 dargestellten Nadelhalter 1 aufsetzbar ist. Zur Verbindung mit dem Nadelhalter weist das Entnahmeröhrchen 7 auf seinem vorderen Ende eine Kappe 8 mit einem darin eingesetzten Stopfen 9 auf. Der Stopfen 9 besteht aus einem weichen, elastischen Kunststoff wie z. b. einem thermoplastischen Elastomer oder aus Kautschuk, wohingegen das Entnahmeröhrchen 7 aus Kunststoff oder Glas, und die Kappe 8 aus einem hinreichend festen Kunststoff gefertigt sind.

Zum Ansetzen an den Nadelhalter 1 wird das aus Kappe 8, Stopfen 9 und dem eigentlichen Entnahmeröhrchen 7 bestehende Behältnis axial von hinten in die Hülse 5 des Nadelhalters 1 eingeschoben. Die Zentrierung erfolgt hierbei über einen zylindrischen Längsabschnitt 10 der Kappe 8, der bündig in die entsprechende Aufnahmebohrung 11 der Hülse 5 einsetzbar ist. Während dieses Einschiebens durchsticht die angespitzte Kanüle 3 den bis dahin unversehrten Stopfen 9 des Behältnisses. Der Gummischlauch 4 wird hierbei an seiner Spitze ebenfalls durchstochen und anschließend in etwa ziehharmonikaförmig zurückgeschoben.

Fig. 3 zeigt Nadelhalter 1 und Entnahmeröhrchen 7 in zusammengesetztem Zustand. In dieser Position werden beide Teile über nachfolgend noch näher beschriebene Verriegelungselemente formschlüssig aneinander gehalten. Infolge des Durchstechens des Stopfens 9 durch die Kanüle 3 besteht eine direkte Strömungsverbindung in das Innere des Entnahmeröhrchens 7. Sofern dieses nach dem Vakuumprinzip arbeitet, d. h. herstellerseitig das Innere unter einen starken Unterdruck gesetzt ist, wird unmittelbar nach dem Durchstich der Kanüle 3 der Unterdruck abgebaut, indem Blut über den Nadelhalter 1 in das Innere des Entnahmeröhrchens 7 einfließt. Dieser Prozeß ist abgeschlossen, sobald der Unterdruck in dem Röhrchen erschöpft ist.

Bei der Ausführungsform nach Fig. 4 hingegen arbeitet das Entnahmebehältnis nicht nach dem Vakuumprinzip, sondern nach dem Aspirationsprinzip. Hierbei ist das Entnahmebehältnis handbetätigt mit einem an der Innenwandung des Entnahmeröhrchens 7 anliegenden Kolben 12, einer Kolbenstange 13 und einem Griffstück 14.

Die Fign. 3 und 4 lassen erkennen, daß der Durchmesser des Nadelhalters 1 nicht größer ist als der größte Durchmesser des Entnahmebehältnisses. Diese relativ schlanke Bauart des Nadelhalters 1 und insbesondere der Hülse 5 des Nadelhalters 1 ist möglich, indem der vordere Teil der Kappe 8 in Gestalt des zylindrischen Längsabschnitts 10 geformt ist, wobei dieser zylindrische Längsabschnitt 10 einen deutlich geringeren Durchmesser aufweist, als ein ebenfalls an der Kappe 8 angeformter Bund 18, mit dem die Kappe den Außenrand des Entnahmeröhrchens 7 umschließt. Bund 18 und Längsabschnitt 10 der Kappe 8 sind über einen sich im wesentlichen radial erstreckenden Absatz 19 einstückig miteinander verbunden. Mittels des Absatzes 19 stützt sich die Kappe 8 gegenüber der Stirnfläche des Entnahmeröhrchens 7 ab. Diese Abstützung erfolgt allerdings nicht unmittelbar, sondern mittelbar unter Zwischenlage eines Kragens 20, welcher einstückig an dem Stopfen 9 angeformt ist. Hierbei befindet sich der Kragen 20 zwischen der Stirnfläche des Entnahmeröhrchens 7 und dem Absatz 19 der Kappe 8. Mit seinem Außenumfang erstreckt sich der Kragen 20 radial bis an die Innenseite 21 des an der Kappe 8 angeformten Bundes 18.

Der von der Kanüle durchstechbare Teil 22 des Stopfens 9 befindet sich deutlich weiter außen, als der Kragen 20. Insbesondere befindet sich der durchstechbare Teil 22 des Stopfens, wie insbesondere Fig. 2 erkennen läßt, innerhalb des zylindrischen Längsabschnitts 10 der Kappe 8 nur geringfügig unterhalb deren äußerer Stirnfläche. In diesem Bereich weist der Stopfen 9 eine kurze Einsenkung 23 auf, in der sich beim Ansetzen an den Nadelhalter 1 der Gummischlauch 4 absetzen und vorläufig zentrieren kann. Zur Verbindung der Kappe 8 mit dem Entnahmeröhrchen 7 sind an der Innenseite 21 des Bundes 18 Rastmittel 24 angeordnet, die in korrespondierende Rastmittel an dem Außenrand des Entnahmeröhrchens 7 eingreifen. In dieser Weise sind Kappe und Entnahmeröhrchen in axialer Richtung formschlüssig miteinander verbunden. Die Rastmittel 24 können auch als Drehentriegelung zum Trennen der Kappe von dem Entnahmeröhrchen ausgebildet sein, eine solche Drehentriegelung ist z. B. in der DE 196 47 978 A1 im einzelnen beschrieben.

Zur Verriegelung des Entnahmebehältnisses an dem Nadelhalter sind an einem äußeren Bund 25 des Nadelhalters über den Umfang verteilt mehrere Nocken 26a angeordnet. Mit diesen Nocken korrespondieren Verriegelungselemente 26b, die um den Längsabschnitt 10 der Kappe 8 herum angeordnet sind. Durch Hintergreifen der Nocken 26a mittels der Verriegelungselemente 26b läßt sich das Entnahmeröhrchen 7 formschlüssig an dem Nadelhalter 1 verriegeln. Auch eine solche Verriegelung, bei der das Entnahmeröhrchen 7 geringfügig gegenüber dem Nadelhalter 1 gedreht werden muß, ist in ihren Einzelheiten in der DE 196 47 978 A1 beschrieben.

In Fig. 2 ist dargestellt, daß auf der Innenseite des Längsabschnitts 10 in Höhe des durchstechbaren Teils 22 des Stopfens 9 Rückhalteelemente 27 angeformt sind. Diese Rückhalteelemente dienen dazu, den Stopfen in axialer Richtung formschlüssig zu fixieren. Hierzu ist der Stopfen 9 in Höhe der Rückhalteelemente 27 mit einer Umfangsnut 28 versehen, in die die Rückhalteelemente nach Art von Widerhaken eingreifen. Bei den Rückhalteelementen 27 kann es sich auch um einen über den Innenumfang geschlossenen Kragen handeln. Sie weisen jeweils eine steile und eine flache rampenförmige Flanke auf, wobei die steilen Flanken zu der Öffnung der Kappe 8, und die flachen Flanken zu dem Entnahmeröhrchen 7 hin weisen.

Zur Herstellung des Entnahmebehältnisses läßt sich der Stopfen 9 von dem Bund 18 der Kappe her in die Kappe einschieben, bis die Rückhalteelemente 27 in die Umfangsnut 28 einrasten. Dieses Einschieben erfolgt gegen den relativ geringen Widerstand der auf dieser Seite flach ansteigenden Flanken der Rückhalteelemente 27. Ein Zurückschieben des Stopfens 9 innerhalb der Kappe 8 ist danach infolge der widerhakenähnlichen Struktur der Rückhalteelemente 27 nicht mehr möglich. Anschließend wird die Kappe 8 mit dem darin bereits befestigten Stopfen 9 auf die Öffnung des Entnahmeröhrchens 7 aufgesetzt, wobei die Rastmittel 24 die Verriegelung beider Teile miteinander sicherstellen. Falls das Entnahmebehältnis nach dem Vakuumprinzip arbeitet, wird vor dem Zusammensetzen noch das Innere des Entnahmeröhrchens 7 evakuiert.

### Bezugszeichenliste

- 1: Nadelhalter
- 2: Nadel
- 3: Kanüle
- 4: Gummischlauch
- 5: Hülse
- 6: Stirnfläche
- 7: Entnahmeröhrchen
- 8: Kappe
- 9: Stopfen
- 10: zylindrischer Längsabschnitt
- 11: Aufnahmebohrung
- 12: Kolben
- 13: Kolbenstange
- 14: Griffstück
- 18: Bund
- 19: Absatz
- 20: Kragen
- 21: Innenseite
- 22: durchstechbarer Teil des Stopfens
- 23: Einsenkung
- 24: Rastmittel
- 25: Bund
- 26a: Nocken
- 26b: Verriegelungselemente
- 27: Rückhalteelement
- 28: Umfangsnut

## Patentansprüche

1. Behältnis für die Entnahme von Proben und insbesondere Blutproben, bestehend aus einem Röhrchen (7) und einer zum Verschließen auf die Öffnung des Röhrchens (7) aufgesetzten Kappe (8), die sich mit einem Absatz (19) gegenüber der die Öffnung umgebenden Stirnfläche des Röhrchens abstützt, die mit einem Bund (18) den Außenrand des Röhrchens umschließt und in die ein gegenüber der Kappe abgedichteter Stopfen (9) aus durchstechbarem und sich selbsttätig wieder verschließendem Material eingesetzt ist, der mit einem einstückig angeformten, umlaufenden Kragen (20) versehen ist, der die Kappe (8) gegenüber dem Röhrchen (7) abdichtet, wobei die Kappe (8) einen von dem Absatz (19) ausgehenden, im wesentlichen zylindrischen Längsabschnitt (10) aufweist, innerhalb dessen sich der durchstechbare Teil (22) des Stopfens (9) befindet,
**dadurch gekennzeichnet,**
**daß** auf der Innenseite des zylindrischen Längsabschnitts (10) in Höhe des durchstechbaren Teils (22) des Stopfens (9) Rückhalteelemente (27) ausgebildet sind, die den Stopfen (9) in axialer Richtung formschlüssig fixieren und daß der durchstechbare Teil (22) des Stopfens nur geringfügig unterhalb der äußeren Stirnfläche der Kappe (8) angeordnet ist.

2. Behältnis nach Anspruch 1, **dadurch gekennzeichnet, daß** sich der Kragen (20) zwischen der Stirnfläche des Röhrchens (7) und dem Absatz (19) der Kappe (8) befindet.

3. Behältnis nach Anspruch 2, **dadurch gekennzeichnet, daß** sich der Kragen (20) radial bis an die Innenseite (21) des an der Kappe (8) angeformten Bundes (18) erstreckt.

4. Behältnis nach einem der Ansprüche 1 bis 3, **dadurch gekennzeichnet, daß** die Kappe (8) mit um den zylindrischen Längsabschnitt (10) herum angeordneten Verriegelungselementen (26b) versehen ist, mittels deren sich das Röhrchen (7) formschlüssig an einem Nadelhalter (1) verriegeln läßt, wobei der zylindrische Längsabschnitt (10) von einer ebenfalls zylindrischen Führungshülse (5) des Nadelhalters (1) aufgenommen wird.

5. Behältnis nach einem der Ansprüche 1 bis 4, **dadurch gekennzeichnet, daß** auf der Innenseite (21) des Bundes (18) Rastmittel (24) ausgebildet sind, die in korrespondierende Rastmittel auf dem Außenrand des Röhrchens (7) eingreifen.

6. Behältnis nach einem der Ansprüche 1 bis 5, **dadurch gekennzeichnet, daß** der Stopfen (9) in Höhe der Rückhalteelemente (27) mit einer die Rückhalteelemente aufnehmenden Umfangsnut (28) versehen ist, und daß die Rückhalteelemente (27) jeweils eine steile und eine flache, rampenförmige Flanke aufweisen, wobei die steilen Flanken zu der Öffnung der Kappe (8) hin weisen.

## Claims

1. Container for taking samples and in particular blood samples, consisting of a tube (7) and a cap (8) which is placed onto the opening of the tube (7) for closure purposes, said cap being supported by a shoulder (19) against the end face of the tube which surrounds the opening, said cap surrounding the outer edge of the tube with a flange (18), into which cap there is inserted a stopper (9) which is made of a perforatable and automatically reclosable material, said stopper being sealed with respect to the cap and being provided with a peripheral collar (20) which is integrally formed in one piece therewith and which seals the cap (8) with respect to the tube (7), wherein the cap (8) has an essentially cylindrical longitudinal section (10) which starts from the shoulder (19) and within which the perforatable part (22) of the stopper (9) is located, **characterized in that** retaining elements (27) are formed on the inner side of the cylindrical longitudinal section (10) at the level of the perforatable part (22) of the stopper (9), which retaining elements fix the stopper (9) in the axial direction in a form-fitting manner, and **in that** the perforatable part (22) of the stopper is arranged only slightly below the outer end face of the cap (8).

2. Container according to Claim 1, **characterized in that** the collar (20) is located between the end face of the tube (7) and the shoulder (19) of the cap (8).

3. Container according to Claim 2, **characterized in that** the collar (20) extends radially up to the inner side (21) of the flange (18) which is integrally formed on the cap (8).

4. Container according to any of Claims 1 to 3, **characterized in that** the cap (8) is provided with locking elements (26b) arranged around the cylindrical longitudinal section (10), by means of which locking elements the tube (7) can be locked to a needle holder (1) in a form-fitting manner, wherein the cylindrical longitudinal section (10) is received by a likewise cylindrical guide sleeve (5) of the needle holder (1).

5. Container according to any of Claims 1 to 4, **characterized in that** latching means (24) are formed on the inner side (21) of the flange (18), which latching means engage in corresponding latching means on the outer edge of the tube (7).

6. Container according to any of Claims 1 to 5, **characterized in that** the stopper (9), at the level of the retaining elements (27), is provided with a peripheral groove (28) which receives the retaining elements, and **in that** the retaining elements (27) each have one steep and one flat, ramp-like flank, wherein the steep flanks point towards the opening in the cap (8).

## Revendications

1. Récipient de prélèvement d'échantillons et en particulier d'échantillons de sang, constitué d'un tube (7) et d'un capuchon (8) placé sur l'ouverture du tube (7) pour la fermer, capuchon qui s'appuie, par un épaulement (19), sur la face frontale du tube qui entoure l'ouverture, qui entoure le bord extérieur du tube par une nervure (18), et dans lequel est inséré un bouchon (9) en matériau transperçable et qui se referme automatiquement, étanche vis-à-vis du capuchon, qui est pourvu d'un collet (20) circonférentiel, formé d'une seule pièce, qui rend le capuchon (8) étanche vis-à-vis du tube (7), le capuchon (8) présentant un tronçon longitudinal (10) sensiblement cylindrique, partant de l'épaulement (19), dans lequel se trouve la partie transperçable (22) du bouchon (9),
**caractérisé en ce que**
sont formés, sur la face interne du tronçon longitudinal (10) cylindrique, à hauteur de la partie transperçable (22) du bouchon (9), des éléments de retenue (27), qui bloquent le bouchon (9) dans la direction axiale, par complémentarité de formes, et **en ce que** la partie transperçable (22) du bouchon n'est disposée que légèrement en-dessous de la face frontale extérieure du capuchon (8).

2. Récipient selon la revendication 1, **caractérisé en ce que** le collet (20) se situe entre la face frontale du tube (7) et l'épaulement (19) du capuchon (8).

3. Récipient selon la revendication 2, **caractérisé en ce que** le collet (20) s'étend radialement jusqu'à la face intérieure (21) de la nervure (18) qui est formée sur le capuchon (8).

4. Récipient selon l'une des revendications 1 à 3, **caractérisé en ce que** le capuchon (8) est pourvu d'éléments de verrouillage (26b) disposés autour du tronçon longitudinal (10) cylindrique, au moyen desquels le tube (7) peut être verrouillé par complémentarité de formes sur un porte-aiguille (1), le tronçon longitudinal (10) cylindrique étant logé dans un manchon de guidage (5) du porte-aiguille (1) qui est également cylindrique.

5. Récipient selon l'une des revendications 1 à 4, **caractérisé en ce que**, sur la face intérieure (21) de la nervure (18) sont formés des moyens d'enclenchement (24) qui s'engagent dans des moyens d'enclenchement correspondants prévus sur le bord extérieur du tube (7).

6. Récipient selon l'une des revendications 1 à 5, **caractérisé en ce que** le bouchon (9), à hauteur des éléments de retenue (27), est pourvu d'une rainure circonférentielle (28) logeant les éléments de retenue, et **en ce que** les éléments de retenue (27) présentent chacun un flanc raide et un flanc plat, les flancs raides étant orientés en direction de l'ouverture du capuchon (8).
